Europäisches Patentamt

European Patent Office

Office européen des brevets

(11) Publication number: **0 247 837**
A2

# EUROPEAN PATENT APPLICATION

(21) Application number: 87304663.5

(51) Int. Cl.⁴: **A 61 F 5/46**

(22) Date of filing: 27.05.87

(30) Priority: 27.05.86 JP 79808/86
20.06.86 JP 94231/86
20.06.86 JP 144610/86

(43) Date of publication of application: 02.12.87
Bulletin 87/49

(84) Designated Contracting States: DE ES FR GB IT SE

(71) Applicant: YUGEN KAISHA HANNMANN,
8-17 Nishigotanda 3-chome, Shinagawa-ku Tokyo-to
(JP)

(72) Inventor: Hannmann, Hisako, 1-21 Sannou 4-chome
Oota-ku, Tokyo-To (JP)

(74) Representative: Heath, Peter William Murray, FRY
HEATH & CO. Seloduct House Station Road, Redhill
Surrey RH1 1PD (GB)

(54) Pessary inserting tool and pessary itself.

(57) A pessary inserting tool (1, 14) has a pessary inserting cylinder (2, 11) and a phushing member (3, 12). The pessary inserting cylinder (2, 11) has a pessary accommodating space (4, 10) into which the pessary (P) is charged in a crushed form. The pushing member (3, 12) is slidably engaged with the pessary accommodating space (4, 10) for pushing out the pessary (P) charged in the space (4, 10). The pessary (P) is provided with a rim (Pr) having a core member (31) therein made of shape memory alloy, super elastic alloy or the like.

## PESSARY INSERTING TOOL AND PESSARY ITSELF

### BACKGROUND OF THE INVENTION

This invention relates to a pessary inserting tool for inserting a pessary into the vagina of a woman and a pessary itself for covering the cervix.

Pessarys are well known as contraceptive appliances which are inserted into the vagina of a woman to cover the cervix in order to prevent semen from entering the womb or uterus. A certain pessary comprises a cup-like diaphragm made of thin natural rubber and a coil-spring rim provided at the periphery of the diaphragm. The pessary covers the cervix in such a manner that the coil-spring rim contacts tightly the vagina wall located at the periphery of the cervix.

In conventional contraceptive pessarys, it is required that they should be fixedly located in the vagina. Accordingly, some women have used the pessarys under the guidance of doctors. Each conventional pessary is inserted into the vagina while being held by two fingers. Therefore, it is difficult to insert the pessary smoothly into the vagina in the case of a Japanese woman whose vagina is relatively small. In addition, Japanese women are, in general, apt to have a feeling of discomfort when they use the pessarys.

Accordingly, in spite of a high reliability in their contraceptive effects, the conventional pessarys are not popular or prevalent because their inserting operation is troublesome and there is a fear of conception or pregnancy because of incompleteness of their inserting operation.

## SUMMARY OF THE INVENTION

It is an object of this invention to provide a pessary inserting tool which can insert a pessary into the vagina so that the cervix is covered correctly with the pessary without using fingers.

It is another object of this invention to provide a pessary which is safe in the vagina and can be easily handled.

According to one aspect of this invention, there is provided a pessary inserting tool for inserting a pessary into the vagina to cover the cervix, which comprises: (a) an inserting cylinder whose front end is in the form of semi-spherical surface, the cylinder having a pessary accommodating space for accommodating the pessary therein in a state wherein a rim of the pessary is crushed in the form of an athletic track, the pessary accommodating space being extended so as to pass through the cylinder in the axial direction of the cylinder; and (b) a pushing member in the form of a piston accommodated slidably in the pessary accommodating space in order to push out the pessary charged in the pessary accommodating space, the pessary discharged from the pessary accommodating space being expanded to cover the cervix.

According to another aspect of this invention, there is provided a pessary for covering the cervix, which comprises: (a) a cup-like diaphragm made of rubber or the like; and (b) a transformable rim located at the periphery of the diaphragm, the periphery of the diaphragm being connected to the rim at a center position on the periphery of the rim in the axial direction of the diaphragm.

The nature, utility, and further features of this invention will be more clearly apparent from the following detailed description with respect to a preferred embodiment of the invention when read in conjunction with the accompanying drawings, briefly described below.

BRIEF DESCRIPTION OF THE DRAWINGS

FIG.1 is a perspective view of a pessary inserting tool according to this invention;

FIG.2 is a front view of the front end of a pessary inserting cylinder;

FIG.3 is a partially vertically sectional view of the pessary inserting cylinder;

FIG.4 is a partially horizontally sectional view of the pessary inserting cylinder;

FIG.5 is a side view of a pushing rod;

FIG.6 is a front view of a piston portion of the pushing rod;

FIG.7 is a horizontally sectional view of the pessary inserting tool, showing a state wherein a pessary is charged in the pessary inserting cylinder;

FIG.8 is a view showing a state wherein the pessary charged in the pessary inserting cylinder is pushed out of the front end of the pessary inserting cylinder;

FIG.9 is a view of a human body, showing a state wherein the pessary is inserted into the vagina;

FIG.10 is a perspective view showing another embodiment of a pessary inserting tool;

FIG.11 is a vertically sectional view of the pessary inserting tool shown in FIG.11;

FIG.12 is a horizontally sectional view of the pessary inserting tool shown in FIG.11;

FIG.13 is a view showing a state wherein the pessary is pushed out of the front end of a pessary inserting cylinder shown in FIG.11;

FIG.14 is a perspective view of a pessary according to this invention;

FIG.15 is a sectional view of a rim of the pessary, taken along the line XV—XV in FIG.14;

FIG.16 is a sectional view of another rim;

FIG.17 is a view showing a core member provided in the rim of the pessary;

FIG.18 is a plan view showing a state wherein the pessary is crushed; and

FIG.19 is a vertically sectional view of a rim of a conventional pessary.

## DETAILED DESCRIPTION OF THE INVENTION

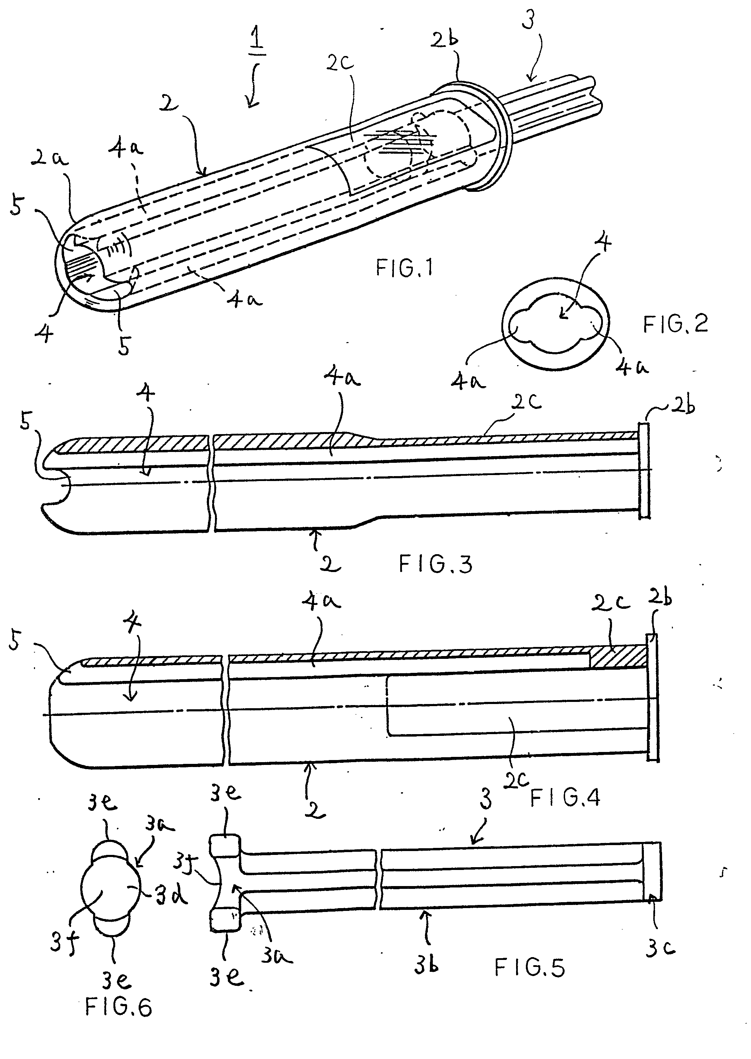

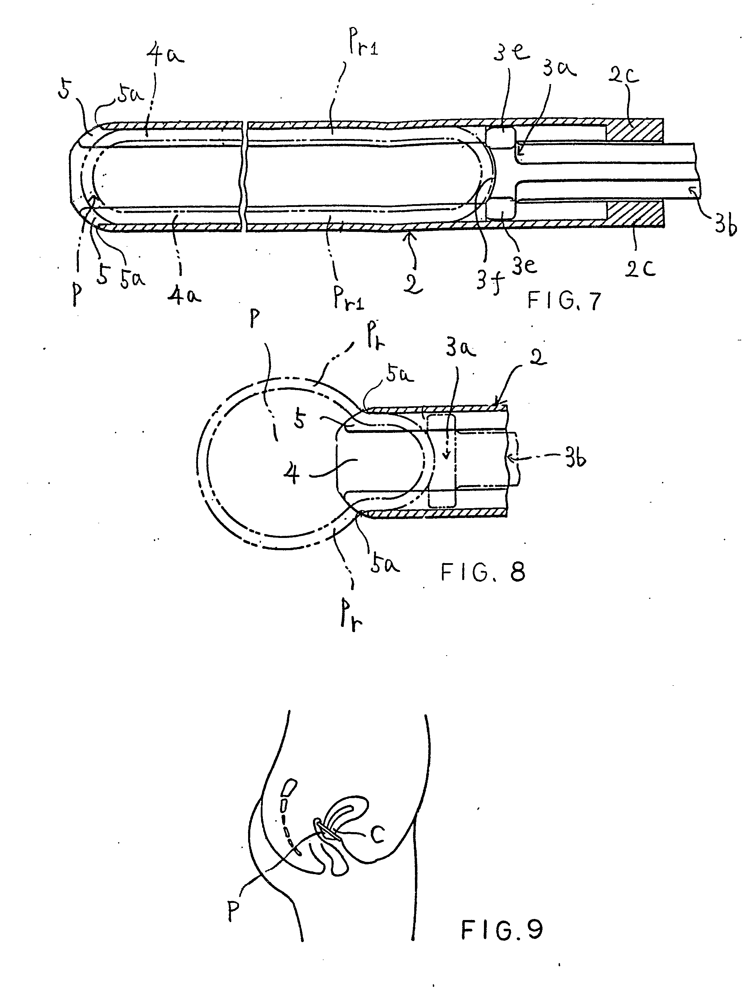

In FIGS 1 to 8, a pessary inserting tool 1 comprises an inserting cylinder 2 which is inserted into the vagina and a pushing rod 3 which functions as a piston for pushing a pessary P (FIG.7). The pessary inserting tool 1 and the pushing rod 3 are made of plastic material or the like.

The inserting cylinder 2 has, in general, a circular shape in cross section and a smooth semi-spherical shape at its front end 2a to prevent the inner wall of the vagina from getting an injury while it has a flange 2b at its root portion. In the pessary inserting cylinder 2 is provided a pessary accommodating space 4 extending through the cylinder 2 in its longitudinal direction, in which the pessary P is accommodated in a state wherein the rim Pr of the pessary P is pushed or crushed inwardly to form a track-like shape for athletics.

The pessary accommodating space 4 has, at its periphery, a pair of guide grooves 4a, 4a provided symmetrically with respect to the longitudinal center axis of the space 4, for guiding the opposite sides of the pessary rim Pr in the form of the track. The two guide grooves 4a extend from the front end of the cylinder 2 to two stopper portions 2c, 2c for preventing the pushing rod 3 from completely coming out of the pessary accommodating space 4, respectively. Each guide groove 4a forms, at its front end, a pessary expanding opening 5 for expanding the pessary P smoothly when the pessary is pushed out of the space 4 into the vagina. Each pessary expanding opening 5 forms a surface arched backward along the semi-spherical surface.

The pushing rod 3 has a piston portion 3a at its front end, a rod portion 3b having a cross-like shape in cross section and a knob portion 3c at its rear end. The piston portion 3a has a circular portion 3d for engaging slidably with the main circular portion of the pessary accommodating space 4 and a pair of semicircular projections 3e, 3e for engaging slidably with the guide grooves 4a, respectively. The circular portion 3d has a concave surface 3f, at its front face, between the two projections 3e. The concave surface 3f contacts a rear part of the pessary rim Pr as shown in FIG.7. The two projections 3e abut against the stopper portions 2c , respectively, when the pushing rod 3 is pulled out in order that the pessary P is charged into the cylinder 2. The tool 1 is arranged in such a manner that the pushing rod 3 is inserted from the front opening of the pessary accommodating space 4 into the cylinder 2, that the rear end of the pushing rod 3 is projected from the rear end of the cylinder 2 and that thereafter the knob portion 3c is attached to the rear end of the pushing rod 3.

On the peripheral surface of the root portion of the cylinder 2 are provided a pair of flat surfaces 2c, 2c symmetrically with respect to the center axis of the cylinder 2. Each flat surface 2c is parallel to a plane including the two guide grooves 4a and functions to show a posture in which the pessary P is accommodated in the cylinder 2. Instead of the flat surfaces 2a, a recess or color portion may be provided to show the posture of the pesssary P.

The operation of this pessary inserting tool 1 will now be explained with reference to FIGS 7 to 9.

Prior to the use of the tool 1, the pessary P is inserted from the front opening of the pessary accommodating space 4 into the cylinder 2 in a state wherein the rim Pr of the pessary P is transformed or crushed into a flat track-like shape so that the linear opposite rim portions Pr1, Pr1 are engaged with the grooves 4a, respectively.

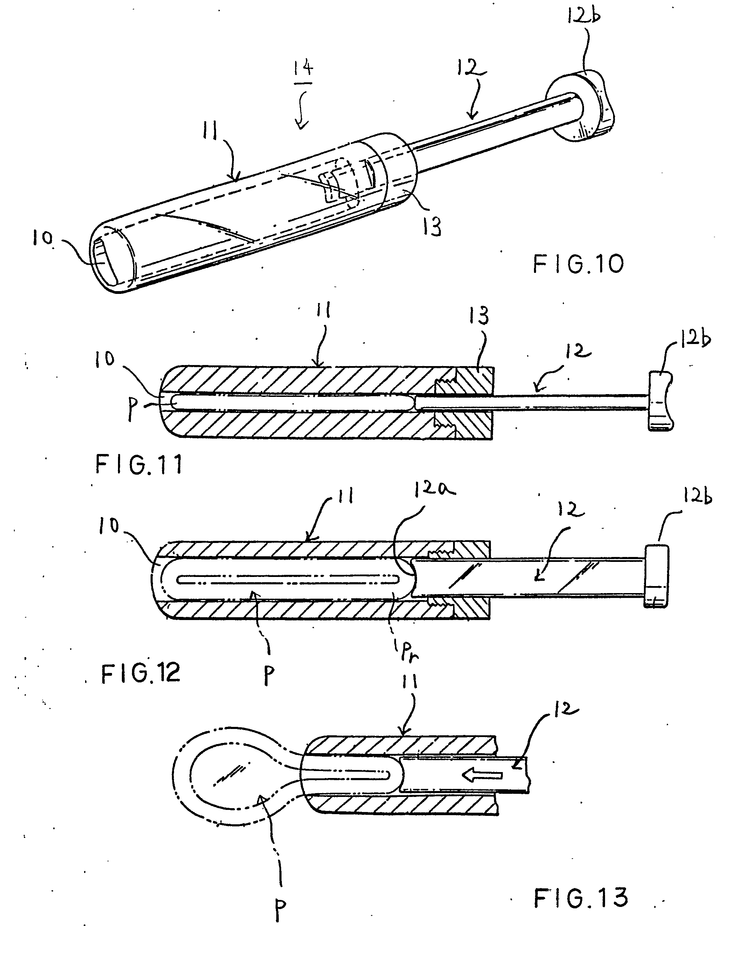

Thereafter, a user inserts the cylider 2 into the vagina while seeing the flat surface 2c on the peripheral surface of the cylinder 2 to confirm the posture of the pessary P. Thereafter, the pushing rod 3 is pushed forward to push out the pessary P from the pessary accommodating space 4. The pessary P is expanded laterally instantly when the rims Pr1 come out of the outer ends 5a of the arched openings 5 due to elasticity of the rim Pr. The cylinder 2 is gradually pulled out of the vagina as the pessary P is pushed out of the openings 5 whereby the pessary P is placed so as to cover the cervix in a state wherein the rim Pr of the pessary P surrounds tightly the periphery of the cervical wall C as shown in FIG.9. This operation can be carried out by one hand. The pessary P is removed from the inside of the vagina by fingers after its use.

As mentioned above, when the pessary P in the accommodating space 4 is partially pushed out , the rim Pr of the pessary P is instantly expanded laterally along the configulation of the arched expanding openings 5. When the pessary P is pushed out completely, the rim Pr becomes a complete circle.

This circular shape facilitates to cover the cervix. Accordingly, even if the cylinder 2 is not inserted in a correct position, the cervix can be reliably covered with the expanded pessary P.

FIGS 10 to 13 show another embodiment of a pessary inserting tool.

In this embodiment, a flat pessary accommodating space 10 is formed in a cylinder 11 and a flat pushing plate 12 is slidably accommodated in the cylinder 11. The pushing plate 12 has, at its front end, a concave face 12a contacting a curved part of the rim Pr of the pessary P and a knob portion 12b at its rear end. The rear end of the cylinder 10 is closed with a closing member 13 through which the pushing plate 12 passes.

The operation of this tool 14 is the same as that of the above tool 1 as shown in FIGS 11 to 13.

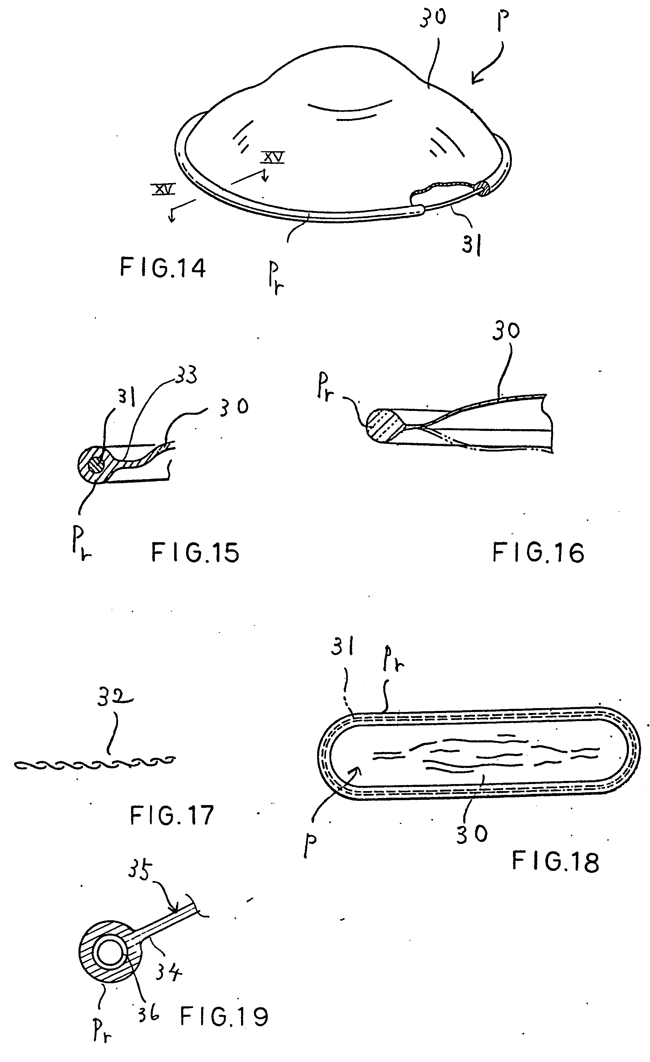

Next, the construction of the pessary P according to this invention will now be explained with reference to FIGS.14 to 18.

The pessary P has a thin diaphragm 30 in the form of a cup which is made of rubber or the like and a rim Pr provided at the periphery of the diaphragm 30. At the center of the rim Pr is accommodated a core member 31 which is made of super-elastic alloy having a property that it can recover even from large deformation in order to increase the ability of restoration (recovery force) to an original circular shape (FIGS.14 and 15). The core member 31 may be made of shape memory alloy (Ni-Ti). The two distal ends of the core member 31 are tightly joined with each other by welding or other joining means to form a circular shape.

In case that shape memory alloy is used for the core member 31, the core member 31 is restored to an original circular shape at temperatures of $35^\circ$ C - $37^\circ$ C corresponding to normal temperatures of a human being. Before the pessary P is charged into the cylinder 2 or 11, it is transformed into a track-like shape and this shape is maintained as shown in FIG.18. Accordingly, the pessary can be easily charged into the cylinder 2 or 11. When the transformed pessary P is pushed into the vagina, it is restored to the original circular shape to cover tightly the cervix. In the case of a pessary with a rim having a core member of shape memory alloy, it can be easily inserted into the vagina without using the inserting tool 1 or 14.

As the core member 31, a wire made of silicon may be used. In addition to this, two wires may be twisted with each other to form an elastic core member 32 as shown in FIG.17.

In the pessary P of this invention, the periphery 33 of the diaphragm 30 is connected to the rim Pr at the center portion of the periphery of the rim Pr in the axial direction of the diaphragm 30 so as to form a shape of a water drop in cross section. Accordingly, it is not necessary to confirm which is the front face of the pessary P, that is, a user can use the pessary P without considering the inside and outside of the diaphragm 30. In a conventional pessary as shown in FIG.19, the periphery 34 of a diaphragm 35 is connected to a deviated upper position of the periphery of a rim Pr which has a coil spring 36 therein. Therefore, in the case of the conventional pessary, a user must consider which is the front surface thereof.

If the rim Pr of the pessary P is made of rubber having a high elasticity, the above core member 31 is not necessary as shown in FIG.16.

11

0247837

CLAIM:

1. A pessary inserting tool (1,14) for inserting a pessary (P) into the vagina to cover the cervix, characlerized in that the pessary inserting tool comprises: an inserting cylinder (2,11) whose front end is in the form of semi-spherical surface, the cylinder (2,11) having a pessary accommodating space (4,10) for accommodating the pessary (P) therein in a state wherein a rim (Pr) of the pessary (P) is crushed in the form of an athletic track, the pessary accommodating space (4,10) being extended so as to pass through the cylinder (2,11) in the axial direction of the cylinder; and a pushing member in the form of a piston accommodated slidably in the pessary accommodating space in order to push out the pessary charged in the pessary accommodating space, the pessary discharged from the pessary accommodating apace being expanded to cover the cervix.

2. A pessary inserting tool according to claim 1, wherein the pessary accommodating space (4) has, at its periphery, a pair of guide grooves (4a) provided symmetrically with respect to the longitudinal center axis of the space (4), the opposite sides of the rim of the pessary (P) transformed into a track-like shape when it is charged into the pessary accommodating space (4) being engaged with the two grooves (4a), respectively, each guide groove (4) forming a pessary expanding opening (5) for expanding laterally the rim (Pr) of the pessary (P) at the front end of the cylinder(4), the pessary expanding opening (5) being in the form of an arched surface along the semi-spherical surface of the front end of the cylinder(2).

3. A pessary inserting tool according to claim 2, wherein the pushing member (3) has a piston portion (3a) at its front end and a rod portion (3b) connected to the piston portion (3a), the piston portion (3a) having a pair of projections (3e) for engaging slidably with the two guide grooves(4a), respectively.

4. A pessary (P) for covering the cervix, which comprises a cup-like diaphragm (30) made of rubber or the like; and a transformable rim (Pr) located at the periphery of the diaphragm(30), charaterized in that the periphery of the diaphragm (30) is connected to the rim (Pr) at a center position on the periphery of the rim (Pr) in the axial direction of the diaphragm(30).

5. A pessary according to claim 4, wherein the rim (Pr) is made of material having a high elasticity.

6. A pessary according to claim 4, wherein the rim (Pr) has a core member (31) therein made of shape memory alloy.

7. A pessary according to claim 4, wherein the rim (Pr) has a core member (31) therein made of super elastic alloy.

FIG.1

FIG.2

FIG.3

FIG.4

FIG.5

FIG.6

FIG. 7

FIG. 8

FIG. 9

0247837

FIG.10

FIG.11

FIG.12

FIG.13

FIG.14

FIG.15

FIG.16

FIG.17

FIG.18

FIG.19